# EUROPEAN PATENT APPLICATION

(11) **EP 4 148 116 A1**
(43) Date of publication of application: **15.03.2023**
(21) Application number: 22175854.3
(22) Date of filing: 27.05.2022
(51) Int. Cl.: C12M 1/32, C12M 1/00, C12M 1/12

(54) **IMAGE CAPTURING IN A CELL CULTIVATION APPARATUS**

(30) Priority: 10.09.2021 EP 21195867
(71) Applicant: Finnadvance Oy, 90220 Oulu (FI)
(72) Inventor: Nguyen, Hoang Tuan, 90220 Oulu (FI); Singh, Prateek, 90220 Oulu (FI)
(74) Representative: Papula Oy

(57) **Abstract**

The present disclosure relates generally to the field of biotechnology and, in particular, to a cell culture apparatus, methods for cell cultivation by using the cell culture apparatus, and a cell culture incubator comprising the cell culture apparatus. The cell culture apparatus comprises a plurality of cell culture modules arranged adjacent to each other. Each cell culture module comprises two or more culture medium reservoirs connected by two or more flow channels. The flow channels go through a basal chamber arranged under an apical chamber. The apical and basal chambers are separated by a porous membrane. The basal chamber has a bottom part arranged higher than a bottom part of each of the culture medium reservoirs. The culture model comprises further a cavity under at least the bottom part of the basal chamber and at least one means for capturing at least one image from a bottom of the at least one cell culture module and/or from a top of the at least one cell culture module.

## Description

### TECHNICAL FIELD

The present disclosure relates generally to the field of biotechnology and image capturing. In particular, the present disclosure relates to a cell culture apparatus, methods for cell cultivation by using the cell culture apparatus, and a cell culture incubator comprising the cell culture apparatus.

### BACKGROUND

Cell culture or cell cultivation involves growing cells of desired type(s) outside a living body under controlled *in vitro* conditions to sustain cell growth and viability. The cell culture is widely used in different biotechnology branches, including cell biology, tissue engineering, biomedical engineering, cell differentiation studies, cell-based biosensors, cell-cell interaction, cell-signaling, cell-migration, physiological and pathophysiological studies, etc.

Environmental conditions created for cultured cells should resemble as closely as possible the conditions experienced by the same cells *in vivo.* This may be done by performing the cell culture in large vessels, such as dishes, spinner and shaker flasks, etc. However, the cell culture conditions provided by these vessels do not truly represent the *in vivo* environment of the cells being cultured. Moreover, since these vessels have a large volume, they consume significant amounts of reagents, culture media, chemicals, etc., thereby making it difficult to control and/or alter the cell culture conditions.

With the advent of microfluidics, new devices and methods configured to cultivate various types of cells, like adherent and non-adherent, under uniform and controlled *in vitro* conditions have been developed. Unlike the conventional cell culture methods based on the above-mentioned vessels, microfluidic cell culture methods may provide continuous nutrient (culture fluid or medium) supply, waste removal, flexibility of schedules, and high automation capability. The less consumption of fluids, their low volumes and therefore the reduced time and cost of cell cultivation make these microfluidic methods particularly interesting for cell-based assays. The main goals of microfluidic cell culture devices are to mimic closely *in vivo* cellular microenvironments and to maintain simplicity for reproducible results.

However, the limiting factors of the current microfluidic cell culture devices are their low throughput and incompatibility with available liquid handling systems and imaging systems due to the discrete arrangement and limited number of microfluidic channels. Furthermore, fluid flow control in the current microfluidic cell culture devices is often limited to one method and not flexible enough to adapt to different resource settings and different cell-based assays. Also, capabilities of the imaging systems to operate in a more optimal way may be further improved.

### SUMMARY

This summary is provided to introduce a selection of concepts in a simplified form that are further described below in the detailed description. This summary is not intended to identify key features of the present disclosure, nor is it intended to be used to limit the scope of the present disclosure.

It is an objective of the present disclosure to provide a technical solution that enables images of a membrane in particular cells grown thereon, resulting high-quality images due to reduced working (i.e. imaging) distance for the means for capturing images.

The objective above is achieved by the features of the independent claims in the appended claims. Further embodiments and examples are apparent from the dependent claims, the detailed description and the accompanying drawings.

According to a first aspect, a cell culture apparatus is provided. The apparatus may comprise a plurality of cell culture modules arranged adjacent to each other. Each cell culture module of the plurality of cell culture modules may comprise at least two culture medium reservoirs, a first chamber, a second chamber, a membrane, at least two flow channels, a cavity, and means for capturing at least one image. Each of the at least two culture medium reservoirs may have a top part and a bottom part, with the top part having an inlet for a culture medium and the bottom part having an outlet for the culture medium. The first chamber may be arranged between the at least two culture medium reservoirs such that the first chamber and the at least two culture medium reservoirs may be aligned with each other in a first direction. The second chamber may be arranged under the first chamber and aligned with the first chamber in a second direction. The second direction may be perpendicular to the first direction. The second chamber may have a bottom part having at least two lateral holes. The bottom part of the second chamber may be arranged higher than the bottom part of each of the at least two culture medium reservoirs. The membrane may have through pores formed therein and may be arranged between the first chamber and the second chamber such that the first chamber and the second chamber are in flow communication with each other via the through pores. Each of the at least two flow channels may connect the outlet of the bottom part of one of the at least two culture medium reservoirs to one of the at least two lateral holes of the bottom part of the second chamber. The cavity may be located at least under the bottom part. The apparatus may further comprise a flow driving unit configured to cause the culture medium to flow, in each cell culture module of the plurality of cell culture modules, between the at least two culture medium reservoirs via the at least two flow channels and the second chamber. The apparatus may also comprise at least one means for capturing at least one image from at least part of a bottom of the at least one cell culture module and/or from at least part of a top of the at least one cell culture module. With this configuration, the cell culture apparatus may cultivate cells of the same or different types on the basal (i.e., bottom) surface of the porous membranes in the cell culture modules under uniform and controlled *in vitro* conditions. Moreover, the deposition of the cells on the basal surface of the porous membranes may be provided without having to invert the apparatus or to use high concentrations of the cells in the culture medium to cause the cells to bond and cover the membrane. A bottom design comprising a cavity may improve flow routing, hold immersion liquid, for example mostly oil and water, and may accommodate sensor elements, for example at least one LED, laser illuminator, diode, photodiode, image intensifier, and/or imager, which may be added for increased functionality. It may also be possible to make high-qualified images of the at least one cell culture medium with the means for capturing images.

According to an example embodiment of the first aspect, each of the at least two flow channels may extend at least partly at a tilting angle to the first direction and the bottom part of the second chamber and at least part of each of the at least two flow channels may form a cavity. With such titled flow channels, it may be possible to provide the cavity to the means for capturing at least one image of the membrane. It may be possible to get images closer, which may improve the image quality. A bent or slope structure on the second chamber or the at least one of the flow channels may facilitate cell deposition on the bottom surface of the membrane without having to invert the whole cell culture module.

According to an example embodiment of the first aspect, each of the at least two flow channels in at least one cell culture module of the plurality of cell culture modules may comprise a first channel portion and a second channel portion. The first channel portion may extend from the bottom part of one of the corresponding culture medium reservoirs and be parallel to the first direction. The second channel portion may connect the first channel portion to the one of the at least two lateral holes of the second chamber. The second channel portion may extend at least partly at the tilting angle to the first direction. The bottom part of the second chamber and at least part of each second channel portions of the at least two flow channels may form the cavity. With this configuration of the flow channels, it is possible to improve flow characteristics in each cell culture module, thereby improving the cell delivery to the second chamber. This may also provide the cavity to the means for capturing images from the membrane.

According to an example embodiment of the first aspect, each of the at least two flow channels in at least one cell culture module may extend at a tilting angle to the first direction starting from the outlet of the corresponding culture medium reservoir. The bottom part of the second chamber and at least part of the each of the at least two flow channels may form the cavity. This means that there may be no first channel portion parallel to the first direction. With such configuration, the flow channels may be easier and cheaper to manufacture.

According to an example embodiment of the first aspect, the tilting angle may fall within a range of 5 degrees to 45 degrees. With such titled flow channels, it may be possible to achieve appropriate flow characteristics and, therefore, improve cell delivery to the second chamber.

According to an example embodiment of the first aspect, each of the at least two flow channels in at least one cell culture module of the plurality of cell culture modules may have a variable or constant channel height; and/or a variable channel width which may increase towards the second chamber. With this configuration of the flow channels, it may be possible to improve flow characteristics in each cell culture module, thereby improving the cell delivery to the second chamber and, consequently, the cell attachment to the basal surface of the porous membrane.

According to an example embodiment of the first aspect, the variable channel width gradually increases towards the second chamber. This may allow achieving better retainment of the laminar flow of the culture medium in the flow channels.

According to an example embodiment of the first aspect, the at least one means for capturing at least one image may be configured to move back and forth in the first direction and capture the at least one image from at least part of the bottom of the at least one cell culture module and/or from at least part of the top of the at least one cell culture module. This may allow the one means for capturing at least one image to be configured to move back and forth in the first direction and capture the at least one image from at least part of the bottom of the whole cell culture plate and/or from at least part of the top of the whole cell culture plate. This may also let capturing images from at least part of the bottom of the at least two flow channels and/or the membrane of the at least one cell culture module of the plurality of cell culture modules. It may also allow capturing images from at least part of the top of the at least one culture medium reservoir, the at least one flow channel, and/or the first chamber of the at least one cell culture module of the plurality of cell culture modules. It may also be possible to capture images on top of the membrane of the at least one cell culture module of the plurality of cell culture modules. Different kind of means for capturing at least one image may be used. Images may also be captured from different perspectives.

According to the example embodiment of the first aspect, at least two cell culture modules are configured to form at least one group. The at least one means for capturing at least one image may be configured to capture the at least one image from at least part of the bottom of the at least two cell culture modules of the at least one group and/or from at least part of the top of the at least two cell culture modules of the at least one group. The group may comprise two or more cell culture modules and less means for capturing images for each group may be needed. This may save costs.

According to the example embodiment of the first aspect, the at least one means for capturing at least one image is configured to move back and forth in the first direction and capture the at least one image from at least part of the bottom of the cell culture module and/or from at least part of the top of the cell culture module. This way images of one cell culture module may be captured from different perspectives and different places.

According to an example embodiment of the first aspect, the at least one means for capturing at least one image may be arranged in the cavity. Thus each cell culture module may comprise own means for capturing at least one image. The image may be captured at least from the membrane. This way it may be possible to obtain better quality images because the means for capturing at least one image may be arranged closer to the membrane.

According to an example embodiment of the first aspect, means for capturing at least one image may be configured to capture at least one optical image. It may be possible to make high-qualified images from the membrane.

According to an example embodiment of the first aspect, means for capturing at least one image may comprise an optical fiber or an objective lens. This may allow to use different solutions when capturing images.

According to an example embodiment of the first aspect, the at least one means for capturing at least one image may be compatible with at least one of the following: an upright and inverted microscope, a digital holography and confocal imaging system, and/or a multiwell plate reader. The apparatus is thus easy to use with other systems.

According to an example embodiment of the first aspect, the bottom part of the second chamber and/or the at least two flow channels may be made of transparent material. With the transparent material quality of the images may be improved.

According to an example embodiment of the first aspect, the first chamber in at least one cell culture module of the plurality of cell culture modules may be implemented as a bottomless hollow tube. With this first chamber, it may be possible to deposit cells on the apical (i.e., top) surface of the porous membrane. The cells deposited on the apical surface of the porous membrane may be of the same or other type compared to the cells deposited on the basal surface of the porous membrane. Thus, the first chamber thus configured may allow one to deposit a cell monolayer on the apical surface of the porous membrane and study the interaction of the cells deposited on the basal and apical surfaces of the membranes. Moreover, with such configuration, the first chamber is easier and cheaper to manufacture.

According to an example embodiment of the first aspect, the first chamber in at least one cell culture module of the plurality of cell culture modules may be implemented as a hollow tube having a curved or angled bottom. In this embodiment, the curved or angled bottom may have at least one cavity and at least one hole formed in each of the at least one cavity. With this first chamber, it may be possible to deposit cells on separate portions of the apical surface of the porous membrane. This configuration of the first chamber may be especially useful for forming similar or different particles under study (e.g., organoids or spheroids) on the apical surface of the porous membrane and study their interaction with the cells deposited on the basal surface of the porous membrane.

According to an example embodiment of the first aspect, the curved or angled bottom of the first chamber may be coated, from outside, with a cell adhesion enhancing layer such that the at least one hole of the at least one cavity may remain open. The cell adhesion enhancing layer may be made of a hydrophilic material (e.g., type I collagen). With such a coating layer, the cells may clump outside the bottom of the first chamber and further adhere onto the apical surface of the porous membrane. Thus, this coating layer allows for less cells to be used in an experiment and more precise deposition of the cells on the apical surface of the porous membrane. In turn, the smaller number of cells also may mean less cell death or, in other words, more healthy cells in the cell culture as dead cells may tend to release factors promoting apoptosis.

According to an example embodiment of the first aspect, the second chamber may have a height falling within a range from 50 µm to 150 µm. By using such a second chamber, it is possible to make the cell culture apparatus according to the first aspect more compact.

According to an example embodiment of the first aspect, the second chamber in at least one cell culture module of the plurality of cell culture modules may be coated, from inside, with a cell adhesion enhancing layer. The cell adhesion enhancing layer may be made of a hydrophilic material. (e.g., type I collagen). With such a coating layer, the cells may attach onto the basal surface of the porous membrane more efficiently. Thus, this coating layer may allow for less cells to be used in an experiment and more precise deposition of the cells on the basal surface of the porous membrane. In turn, the smaller number of cells also may mean less cell death or, in other words, more healthy cells in the cell culture as dead cells may tend to release factors promoting apoptosis.

According to an example embodiment of the first aspect, each of the at least two flow channels in at least one cell culture module of the plurality of cell culture modules may be coated, from inside, with a cell adhesion enhancing layer. The cell adhesion enhancing layer may be made of a hydrophilic material (e.g., type I collagen). By using the adhesion enhancing layer, cell attachment onto the sides of the flow channels (as well as on the sides of the second chamber and the porous membrane) may become stronger, cell proliferation may be appropriate to a tissue being modelled, and cell viability may be better. Selecting the correct adhesion enhancing layer may also play a role in the proper communication between cell types. Thus, this coating layer may allow for less cells to be used in an experiment and more precise deposition of the cells on the basal surface of the porous membrane. In turn, the smaller number of cells may also mean less cell death or, in other words, more healthy cells in the cell culture as dead cells may tend to release factors promoting apoptosis.

According to an example embodiment of the first aspect, the flow driving unit may be configured to cause the culture medium to flow, in each cell culture module of the plurality of cell culture modules, between the at least two culture medium reservoirs by:
- supplying a compressed gas or a pressurized air to the inlet of each of the at least two culture medium reservoirs; or
- pipetting the culture medium from one of the at least two culture medium reservoirs to another of the at least two culture medium reservoirs at regular time intervals or based on a level of the culture medium in each of the at least two culture medium reservoirs; or
- periodically rocking the plurality of cell culture modules.

Thus, unlike the current microfluidic cell culture devices, the cell culture apparatus according to the first aspect may be compatible with different flow control means, i.e., pneumatic pump-actuated flow control, and pumpless flow control (which is provided by means of gravity as a result of rocking the plurality of cell culture modules or by means of culture medium pipetting).

According to an example embodiment of the first aspect, each of the at least two culture medium reservoirs in at least one cell culture module of the plurality of cell culture modules may be implemented as a hollow tube. With such configuration, the culture medium reservoirs may be easier and cheaper to manufacture.

According to an example embodiment of the first aspect, the bottom part of each of the at least two culture medium reservoirs may have a positively tapered profile. By using such a configuration of the culture medium reservoirs, it may be possible to cause the whole culture medium contained in the culture medium reservoirs to flow to the bottom towards the second chamber even when the apparatus is tilted, so that no culture medium may be left in the culture medium reservoirs. This also may help to reduce the number of cells that may be needed to be deposited or grown on the basal surface of the porous membrane. Additionally, this configuration of the culture medium reservoirs may allow one to achieve optimal flow rates in each cell culture module.

According to an example embodiment of the first aspect, at least one cell culture module of the plurality of cell culture modules further may comprise a first electrode arranged in the first chamber and a second electrode arranged in the second chamber. These electrodes may serve different purposes. For example, they may be used to feed electrical pulses to muscle or nerve cells present in the first and second chambers or be coupled to other sensors of various types. More specifically, these electrodes may be used to perform real-time Transepithelial/Transendothelial Electrical Resistance (TEER) measurements; in this case, signals may be read out by an external control unit.

According to an example embodiment of the first aspect, at least one cell culture module of the plurality of cell culture modules may further comprise a first electrode arranged in one or more of the at least two culture medium reservoirs and a second electrode arranged in the first chamber. This arrangement of the first and second electrodes may be also used to perform the TEER measurements, for example.

According to an example embodiment of the first aspect, at least one cell culture module of the plurality of cell culture modules may further comprise at least two third electrodes arranged in the first chamber. The third electrodes may be arranged on the same wall or on opposite walls inside the first chamber. These electrodes may be used to perform liquid/medium level measurements in the first chamber.

According to an example embodiment of the first aspect, at least one cell culture module of the plurality of cell culture modules may further comprise at least two fourth electrodes imbedded into the membrane. These electrodes may be used, for example, to provide different stimulus signals to the cells deposited on the apical and/or basal surface of the membrane.

According to an example embodiment of the first aspect, at least one cell culture module of the plurality of cell culture modules may further comprise at least one of the following: an oxygen sensor, a pH sensor, and/or a CO2 sensor in the first chamber and/or the second chamber. These sensors may enable real-time measurements of parameters crucial for cell behavior.

According to an example embodiment of the first aspect, the through pores of the membrane may have an average pore size falling within a range from 0.2 µm to 10 µm. By varying the average pore size within this range, it may be possible to study the behavior of cells of different sizes, which may be deposited on one or each of the apical and basal surfaces of the membrane.

According to a second aspect, a cell culture incubator is provided. The cell culture incubator may comprise the cell culture apparatus according to the first aspect, and a pipetting station configured to feed the culture medium to each of the at least two culture medium reservoirs in each cell culture module of the plurality of cell culture modules. With this configuration, the cell culture incubator may cultivate cells of the same or different types on the porous membranes of the cell culture modules under unform and controlled *in vitro* conditions. Moreover, the deposition of the cells on the basal surface of the porous membranes may be provided without having to invert the cell culture apparatus or to use high concentrations of the cells in the culture medium to cause the cells to bond and cover the membrane.

According to a third aspect, a method for cell cultivation by using the cell culture apparatus according to the first aspect is provided. The method may start with providing the culture medium to one of the at least two medium reservoirs in each cell culture module of the plurality of cell culture modules. Then, the method may proceed to cause, by the flow driving unit, the culture medium to flow from said one of the at least two culture medium reservoirs to the rest of the at least two culture medium reservoirs in each cell culture module of the plurality of cell culture modules for a predefined time period. The predefined time period may be selected based on the culture medium. By so doing, it may be possible to cultivate cells of the same or different types on the porous membranes of the cell culture modules under unform and controlled *in vitro* conditions. Moreover, the deposition of the cells on the basal surface of the porous membranes may be provided without having to invert the cell culture apparatus or to use high concentrations of the cells in the culture medium to cause the cells to bond and cover the membrane.

According to an example embodiment of the third aspect, the method may further comprise capturing at least one image from at least one e cell culture module of the plurality of cell culture modules by the at least one means for capturing at least one image. With this method it may be possible to make high-qualified images at least one e cell culture module.

According to an example embodiment of the third aspect, the method may further comprise, when causing the culture medium to flow, feeding another culture medium via the first chamber towards the membrane in at least one cell culture module of the plurality of cell culture modules. By so doing, it may be possible to cultivate a first type of cells on the basal surface of the porous membranes and a different second type of cells on the apical surface of the porous membranes, thereby generating different cell co-cultures.

According to an example embodiment of the third aspect, the culture medium may be caused to flow by applying a positive pressure to the at least two culture medium reservoirs in each cell culture module of the plurality of cell culture modules for the predefined time period. By so doing, it is possible to provide a proper culture medium flow in each cell culture module, thereby improving cell cultivation on the porous membranes in each cell culture module.

According to an example embodiment of the third aspect, the culture medium may be caused to flow by applying a positive pressure to said one of the at least two culture medium reservoirs in each cell culture module of the plurality of cell culture modules for the predefined time period, while capping the rest of the at least two culture medium reservoirs.

By so doing, it may be possible to provide a proper culture medium flow in each cell culture module, thereby improving cell cultivation on the porous membranes in each cell culture module.

According to an example embodiment of the third aspect, the at least two culture medium reservoirs in each cell culture module of the plurality of cell culture modules may comprise a first culture medium reservoir and a second culture medium reservoir. In this embodiment, the culture medium may be caused to flow by:
- applying a first positive pressure to the first culture medium reservoir in each cell culture module of the plurality of cell culture modules for the predefined time interval, while capping the second culture medium reservoir; and
- applying a second positive pressure to the second culture medium reservoir in each cell culture module of the plurality of cell culture modules for the predefined time interval, while capping the first culture medium reservoir.

By so doing, it may be possible to provide a proper culture medium flow in each cell culture module, thereby improving cell cultivation on the porous membranes in each cell culture module.

According to a fourth aspect, a method for cell cultivation by using the cell culture apparatus according to the first aspect is provided. The method may start with providing the culture medium to one or more of the at least two medium reservoirs in each cell culture module of the plurality of cell culture modules. Then, the method may proceed to cause, by the flow driving unit, the culture medium to flow to the second chamber from said one or more of the at least two medium reservoirs in each cell culture module of the plurality of cell culture modules. After that, the cell culture apparatus may be placed in an inverted position. Next, the method may proceed to incubate the cell culture apparatus in the inverted position for a predefined time period. The predefined time period may be selected based on the culture medium. By so doing, it may be possible to cultivate cells of the same or different types on the porous membranes of the cell culture modules under unform and controlled *in vitro* conditions. Although the deposition of the cells on the basal surface of the porous membranes may be provided by inverting the cell culture apparatus, the method according to the fourth aspect does not require high concentrations of the cells to be used in the culture medium to cause the cells to bond and cover the membrane.

According to an example embodiment of the fourth aspect, the method may further comprise, when causing the culture medium to flow, another culture medium may be feed via the first chamber towards the membrane in at least one cell culture module of the plurality of cell culture modules. By so doing, it may be possible to cultivate a first type of cells on the basal surface of the porous membranes and a different second type of cells on the apical surface of the porous membranes, thereby generating different cell co-cultures.

According to an example embodiment of the fourth aspect, the culture medium may comprise human brain vascular endothelial cells and said another culture medium may comprise human astrocytes. By using these types of cells, it may be possible to create artificial blood-brain barriers (BBBs) or create human mimicking artificial BBBs.

Other features and advantages of the present disclosure will be apparent upon reading the following detailed description and reviewing the accompanying drawings.

### BRIEF DESCRIPTION OF THE DRAWINGS

The present disclosure is explained below with reference to the accompanying drawings in which:
FIG. 1A shows a block diagram of a cell culture apparatus in accordance with one exemplary embodiment;
FIG. 1B shows a block diagram of a cell culture plate in accordance with one exemplary embodiment;
FIGs. 2A and 2B show different schematic views of a cell culture module included in the apparatus shown in FIG. 1A in accordance with a first exemplary embodiment, namely: FIG. 2A shows a schematic side view of the cell culture module and FIG. 2B shows a schematic top view of the cell culture module;
FIG. 3 shows a block diagram of a cell culture incubator in accordance with one exemplary embodiment;
FIG. 4 shows a flowchart of a method for cell cultivation by using the apparatus shown in FIG. 1A in accordance with a first exemplary embodiment;
FIG. 5 shows a flowchart of a method for cell cultivation by using the apparatus shown in FIG. 1A in accordance with a second exemplary embodiment; and
FIGs. 6A and 6B show confocal images of a BBB model by using the apparatus shown in FIG. 1A in accordance with the method shown in FIG. 5.

### DETAILED DESCRIPTION

Various embodiments of the present disclosure are further described in more detail with reference to the accompanying drawings. However, the present disclosure may be embodied in many other forms and should not be construed as limited to any certain structure or function discussed in the following description. In contrast, these embodiments are provided to make the description of the present disclosure detailed and complete.

According to the detailed description, it will be apparent to the ones skilled in the art that the scope of the present disclosure encompasses any embodiment thereof, which is disclosed herein, irrespective of whether this embodiment is implemented independently or in concert with any other embodiment of the present disclosure. For example, apparatuses and/or methods disclosed herein may be implemented in practice using any numbers of the embodiments provided herein. Furthermore, it should be understood that any embodiment of the present disclosure may be implemented using one or more of the elements presented in the appended claims.

The word "exemplary" is used herein in the meaning of "used as an illustration". Unless otherwise stated, any embodiment described herein as "exemplary" should not be construed as preferable or having an advantage over other embodiments.

Any positioning terminology, such as "left", "right", "top", "bottom", "above", "under", "apical", "basal", etc., may be used herein for convenience to describe one element's or feature's relationship to one or more other elements or features in accordance with the figures. It should be apparent that the positioning terminology is intended to encompass different orientations of the structure and device disclosed herein, in addition to the orientation(s) depicted in the figures. As an example, if one imaginatively rotates the structure or device in the figures 90 degrees clockwise, elements or features described as "top" and "bottom" relative to other elements or features would then be oriented, respectively, "right" and "left" relative to the other elements or features. Therefore, the positioning terminology used herein should not be construed as any limitation of the present disclosure.

Furthermore, although the numerative terminology, such as "first", "second", etc., may be used herein to describe various embodiments, elements or features, it should be understood that these embodiments, elements or features should not be limited by this numerative terminology. This numerative terminology is used herein only to distinguish one embodiment, element or feature from another embodiment, element or feature. For example, a first chamber discussed below could be called a second chamber, and vice versa, without departing from the teachings of the present disclosure.

In the embodiments disclosed herein, a cell may refer to a biological cell, such as a plant cell, an animal cell (e.g., a mammalian cell), a bacterial cell, a fungal cell, or the like. A mammalian cell may be, for example, from a human, a mouse, a horse, a goat, a sheep, a cow, a primate, or the like.

As used in the embodiments disclosed herein, a culture medium, also referred to as a growth medium, may refer to a liquid, suspension, or gel designed to support cellular growth in an artificial environment, i.e., *in vitro.* There are different types of culture media suitable for growing different types of cells. In general, the culture media may be broken into two primary categories: natural media and synthetic media. The natural media are those that are derived from tissue extraction or animal body fluids, such as plasma, lymph, and serum. The synthetic media are those created using a variety of organic and inorganic compounds. Moreover, the culture medium itself may comprise cells, bodies of cells (e.g., organoids), lipid particles, including natural or engineered (e.g., exosomal microvesicles, vacuoles, micelles or lipid particles of various design, virus particles, nanoparticles, etc.).

In the embodiments disclosed herein, a cell culture apparatus may refer to an apparatus having various wells (e.g., reservoirs, chambers, etc.) connected by microchannels in which fluids (i.e., culture media) will exhibit microfluidic behavior in their flow through the microchannels. Such a cell culture apparatus is also referred to as a microfluidic chip in this technical field. The microfluidic cell culture performed by the cell culture apparatus is generally related to cell culture, maintenance and perturbation in micro-scale fluid volumes. The reasons behind the popularity of the microfluidic cell culture are both economic and scientific. The microfluidic chips have the advantages of *in vitro* cell culture (high-throughput, parallel experiments, experiments may be done at the discretion of an experimenter, no need for specialized infrastructure and personnel, etc.) with *in vivo* like performance. For example, in mouse models, drugs are really selected for mice, not humans. By using human cells, drugs are screened for humans. Thus, using humanized microfluidic cell and tissue cultures to screen drug candidates reduces pre-clinical trial time and those drugs entering clinical testing are better suited for humans. This may reduce the probability of adverse effects and increase the chance of showing efficacy resulting in less failures in clinical trials.

In the embodiments disclosed herein, each microchannel of the cell culture apparatus is also referred to as a flow channel and may relate to a sub-millimeter-scale channel that has a hydraulic diameter below 1 mm and is used for fluidically connecting the wells of the cell culture apparatus. In other words, the microchannel or flow channel generally denotes a channel configured to pass different fluids, such, for example, as culture media (e.g., cell suspensions), reagents, or gels, in micro-scale volumes. The microchannel may be shaped such that it has appropriate flow characteristics (e.g., flow rates) depending on particular applications. For example, the microchannel may have a rectangular, e.g., square, or rounded cross-section, as well as be straight, bended, or tilted towards a required direction.

The microfluidic cell culture may involve using a porous membrane sandwiched between two flow layers within a microchannel or between a culture chamber and a microchannel for cell deposition. However, the membrane-based cell culture apparatuses known so far suffer from low throughput and incompatibility with available liquid handling systems (e.g., microtiter plate formats) and imaging systems. Furthermore, the flow control in such apparatuses is often limited to one method and not flexible enough to adapt to different resource settings. On top of that, the cell deposition on the basal (i.e., bottom) surface of the porous membrane(s) is possible in the current cell culture apparatuses only with very high cell concentrations, liquid flow control, and by inverting the cell culture apparatus.

The exemplary embodiments disclosed herein provide a technical solution that allows mitigating or even eliminating the drawbacks of the prior art. In particular, the technical solution disclosed herein provides a cell culture apparatus comprising a plurality of cell culture modules arranged adjacent to each other. Each cell culture module may comprise two or more culture medium reservoirs connected by two or more flow channels. The flow channels go through a basal chamber arranged under an apical chamber. The apical and basal chambers are separated by a porous membrane. The basal chamber has a bottom part arranged higher than a bottom part of each of the culture medium reservoirs. Each cell culture module may further comprise a cavity under at least the bottom part of the second chamber. In this apparatus configuration, flows of culture media may be perfused and regulated between the culture medium reservoirs in each cell culture module by either placing the apparatus on a rocking platform or connecting the apparatus to a pneumatic pump. Further the apparatus may comprise at least one means for capturing at least one image from at least part of a bottom of the at least one cell culture module and/or from at least part of a top of the at least one cell culture module. The means for capturing at least one image may also be arranged in the cavity and configured to capture images from the membrane. Moreover, the apparatus thus configured may cultivate cells of the same or different types on the basal surface of the porous membranes in the cell culture modules under uniform and controlled *in vitro* conditions. The deposition of the cells on the basal surface of the porous membranes may be provided without having to invert the apparatus and to use high concentrations of the cells in the culture medium.

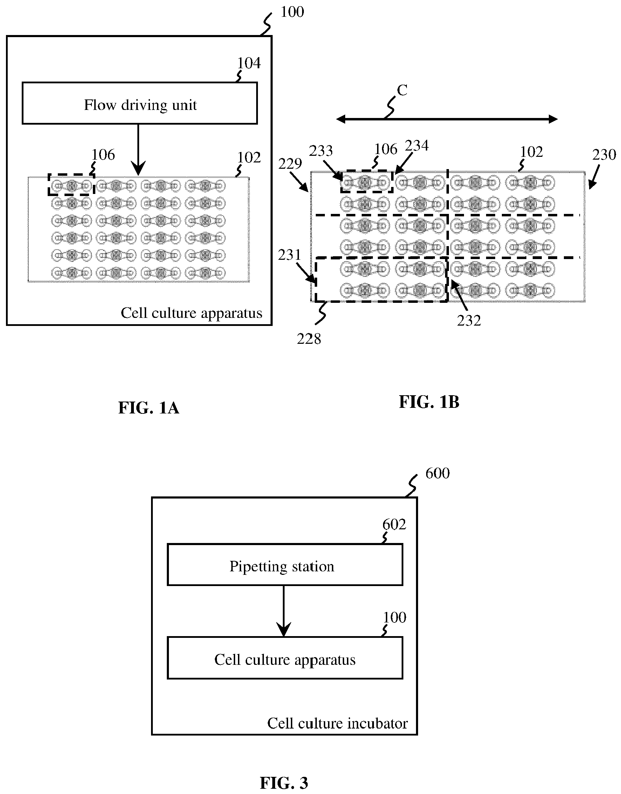

FIG. 1A shows a block diagram of a cell culture apparatus 100 in accordance with one exemplary embodiment. The apparatus 100 is intended for the above-mentioned microfluidic cell culture. As shown in FIG. 1A, the apparatus 100 may comprise a cell culture plate 102 and a flow driving unit 104 coupled to the cell culture plate 102. The cell culture plate 102 may comprise multiple cell culture modules 106 and may be configured to fit the standard 96, 384, or 1536 microtiter plates. The flow driving unit 104 may be configured to cause a culture medium to flow in each cell culture module 106, as will be described below in more detail. It should be noted that the number, arrangement, and interconnection of the constructive elements constituting the apparatus 100, which are shown in FIG. 1A, are not intended to be any limitation of the present invention, but merely used to provide a general idea of how the constructive elements may be implemented within the apparatus 100. For example, the cell culture plate 102 may be replaced with two or more cell culture plates, and the flow driving unit 104 may be replaced with two or more flow driving units each configured to control a culture medium flow in one of the cell culture plates.

FIG. 1B shows a block diagram of a cell culture plate 102 of a cell culture apparatus 100 in accordance with one exemplary embodiment. The cell culture plate may be divided into at least one group 228. The at least one group 228 may comprise at least two cell culture modules 106. The example of FIG. 1B shows six groups 228, wherein each group 228 comprise four cell culture modules 106. It is clear that different amount of groups may be used, and each group may comprise a different number of cell culture modules 106.

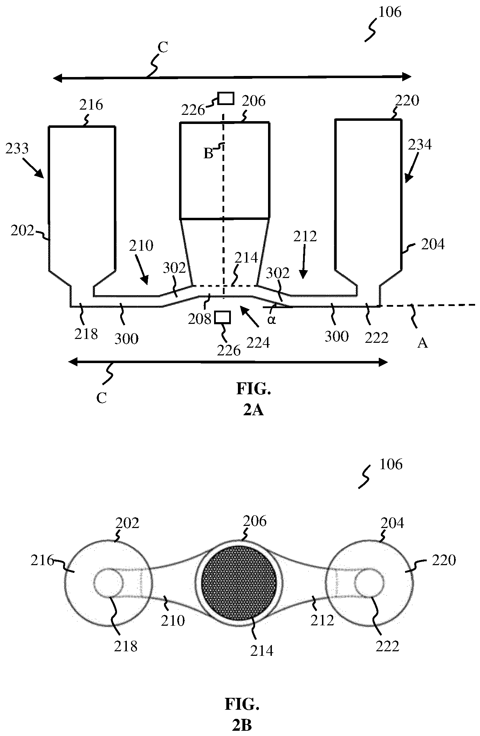

FIGs. 2A and 2B show different schematic views of the cell culture module 106 included in the apparatus 100 in accordance with a first exemplary embodiment. More specifically, FIG. 2A shows an example of schematic side view of the cell culture module 106 and FIG. 2B shows an example of a schematic top view of the cell culture module 106. As shown in FIGs. 2A and 2B, the cell culture module 106 may comprises a first culture medium reservoir 202, a second culture medium reservoir 204, a first (apical or top) chamber 206, a second (basal or bottom) chamber 208, a first flow channel 210, a second flow channel 212, a membrane 214, a cavity 224 below the second chamber 208, and at least one means for capturing at least one image 226. It should be again noted that the present disclosure is not limited to the shown number, arrangement, and interconnection of the constructive elements of the cell culture module 106. According to an example embodiment, there may be only one means for capturing at least one image 226. According to another example embodiment, there may be several means for capturing at least one image 226. In some example embodiments, there may be more than two medium culture reservoirs, thereby leading to more than two flow channels. In other example embodiments, there may be more than two apical chambers and more than two basal chambers between the two culture reservoirs, thereby also increasing the number of the flow channels connecting the two culture reservoirs through the apical chambers and the basal chambers.

The first culture medium reservoir 202 may have a top part with an inlet 216 for the culture medium and a bottom part with an outlet 218 for the culture medium. Similarly, the second culture medium reservoir 204 may have a top part with an inlet 220 for the culture medium and a bottom part with an outlet 222 for the culture medium. The first and second culture medium reservoirs 202 and 204 may be implemented as identical hollow tubes. Although FIGs. 2A and 2B show that each of the first and second culture medium reservoirs 202 and 204 has a circular cross-section, this should not be construed as any limitation of the present disclosure; in some embodiments, any other cross-sectional shapes, such as polygonal, oval, etc., for example, are possible, if required and depending on particular applications. Moreover, the bottom part of each of the first and second culture medium reservoirs 202 and 204 may have a different profile, for example, a positively tapered profile as shown in FIGs. 2A and 2B.

The first chamber 206 may be arranged between the first and second medium culture reservoirs 202 and 204 such that the first chamber 206 and the first and second culture medium reservoirs 202 and 204 are aligned with each other in a first (horizontal) direction A. The first chamber 206 may be implemented as a bottomless hollow tube, for example, with a positively tapered profile (as shown in FIG 2A) or with a uniform cross-section. Again, the shown circular cross-section of the first chamber 206 is for illustrative purposes only and should not be considered as any limitation of the present disclosure.

The second chamber 208 may be arranged under the first chamber 206 and aligned with the first chamber 206 in a second (vertical) direction B. The first direction A may be approximately perpendicular to the second direction. An angle between the first and the second direction A, B is about 90 degrees, for example. The second chamber 208 may have a cross-section corresponding to the cross-section of the first chamber 206. The second chamber 208 may have a bottom part having two or more lateral holes (not shown in FIG 2A). As shown in FIG 2A, the second chamber 208 may have dimensions significantly smaller than the first chamber 206. For example, if the first chamber have a height of about 7 mm, then the height of the second chamber 208 may fall within a range from 50 µm to 150 µm (at height values higher than 150 µm, the second chamber 208 may lose microfluidic properties). Such height values of the first and second chambers 206 and 208 (in addition to the heights of the reservoirs 202 and 204) may make the cell culture module 106 more compact, thereby reducing the total dimensions of the apparatus 100.

The membrane 214 may have through pores (see FIG. 2B) and may be arranged between the first chamber 206 and the second chamber 208 such that the first chamber 206 and the second chamber 208 are in flow communication with each other via the through pores of the membrane 214. The membrane 214 may be composed of silicone, plastic, protein, natural polymers, artificial polymers (e.g., polyester (PET)), metallic polymers or carbohydrates (e.g., cellulose), and may be formed by using one of the following methods: lithography, stamping, casting, electrospinning or *in situ* polymerization. The through pores may have different cross-sectional shapes, such, for example, as triangular, rectangular, square, oval, or polygonal (e.g., hexagonal). The polygonal cross-section shape of the through pores may refer to a convex or concave polygon. In some other embodiments, the through pores may have different cross-sectional shapes, such as any combination of two or more of the above-mentioned cross-sectional shapes (e.g., the combination of circular and square cross-section shapes). Furthermore, each of the through pores may have a pore size varying within a predefined range of values. For example, the pore size may vary from 0.2 µm to 10 µm. In general, the predefined range of values for the pore size (as well as the spacing between the through pores) may be selected based on certain cells to be deposited on the membrane 214. More specially, the pore sizes may be more or less than sizes of the cells to be deposited on the membrane. For example, if the pore size is less than the cell size, then it is possible to prevent the cells from migrating between the basal and apical surfaces of the membrane 214.

The first and second flow channels 210 and 212 may be microchannels providing the passage of the culture medium between the first and second culture medium reservoirs 202 and 204 through the second chamber 208. In particular, the first flow channel 210 may connect the outlet 218 of the bottom part of the first culture medium reservoir 202 to one or more of the lateral holes arranged on the left side of the bottom part of the second chamber 208. The second flow channel 212 may connect the outlet 222 of the bottom part of the second culture medium reservoir 204 to one or more of the lateral holes arranged on the right side of the bottom part of the second chamber 208. Each of the first and second flow channels 210 and 212 may have a longitudinal section and a cross-section which allow one to achieve required flow characteristics (e.g., an appropriate flow rate). For example, each of the first and second flow channels 210 and 212 may have a variable channel height and a variable channel width which increase (e.g., gradually) towards the second chamber 208. In an example of FIG. 2A each of the first and second flow channels 210 and 212 have a constant channel height and a variable channel width which increase (e.g., gradually) towards the second chamber 208. According to an example embodiment, each of the at least two flow channels 210, 212 in at least one cell culture module 106 of the plurality of cell culture modules may have a variable or constant channel height, and/or a variable channel width which increase towards the second chamber 208.

In an example of FIG. 2A can be seen a first and second flow channel 210, 212 of the cell culture module 106. As shown in FIG. 2A, the second flow channel 212 may comprise a first channel portion 300 and a second channel portion 302. It should be noted that the first flow channel 210 may have a similar first channel portion 300 and a second channel portion 302 as the second flow channel, only presented as a mirror image. The first channel portion 300 may extend from the outlet 222 of the bottom part of the second culture medium reservoir 204 and is parallel to the first (horizontal) direction. The second channel portion 302 may connect the first channel portion 300 to the lateral hole(s) (not shown in FIG. 2A) arranged on the right side of the second chamber 208. The second channel portion may be tilted up (i.e., at a tilting angle α to the first direction) such that the bottom part of the second chamber 208 may be arranged or lifted higher than the bottom part of the second culture medium reservoir 204 (i.e., higher than the end of the outlet 222). The tilting angle α of the second channel portion 302 relative to the first (horizontal) direction A may preferably fall within a range of 5 degrees to 45 degrees to provide appropriate flow characteristics.

According to another example embodiment, each of the first and second flow channels 210 and 212 may be fully tilted up at the tilting angle α, starting from the outlet of the corresponding culture medium reservoir 218, 222 (i.e., there may be no first channel portion parallel to the first (horizontal) direction). According to an example embodiment, each of the at least two flow channels 210, 212 in at least one cell culture module 206 extends at a tilting angle α to the first direction A starting from the outlet 218, 222 of the corresponding culture medium reservoir 202, 204. The bottom part of the second chamber 208 and at least part of the each of the at least two flow channels 210, 212 may form a cavity.

According to an example embodiment, each of the at least two flow channels 210, 212 in at least one cell culture module 106 of the plurality of cell culture modules comprising a first channel portion 300 and a second channel portion 302. The first channel portion 300 may extend from the bottom part of one of the corresponding culture medium reservoir 202, 204 and being parallel to the first direction A. The second channel portion 302 may connect the first channel portion 300 to the one of the at least two lateral holes of the second chamber 208. The second channel portion may extend at least partly at the tilting angle α to the first direction A. The bottom part of the second chamber 208 and each of the second channel portions 302 of the at least two flow channels 210, 212 may form the cavity 224 under the lifted bottom part of the second chamber 208. The cavity 224 may be inverted.

An example of FIG. 2A shows that the culture apparatus 100 further comprises at least one means for capturing at least one image 226 from at least part of a bottom of the at least one cell culture module 106 and/or from at least part of a top of the at least one cell culture module 106. According to an example embodiment, the at least one means for capturing at least one image 226 is configured to move back and forth in the first direction A and capture the at least one image from at least part of the bottom of the at least one cell culture module 106 and/or from at least part of the top of the at least one cell culture module 106. The means for capturing images 226 may take one image, series of images, and/or video from one or more locations under, above, and/or in the cavity 224 of the at least one cell culture module 106. Moving of the means for capturing at least one image 226 is shown in an example embodiment of FIG. 1B and 2A with a two-way arrows C, which is parallel to the first direction A. The means for capturing at least one image 226 may be a camera, a video device, or any other imaging device.

According to an example embodiment, there may be one or more means for capturing images 226 and they may be located below the cell culture module 106, above the cell culture module 106 and/or in the cavity of the cell culture module 106. This may allow capturing images from at least part of the bottom of the at least two flow channels 210, 212 and/or the membrane 214 of the at least one cell culture module 106 of the plurality of cell culture modules. It may also allow capturing images from at least part of the top of the at least one culture medium reservoir 202, 204, the at least one flow channel 210, 212, the membrane 214, and/or the first chamber 206 of the at least one cell culture module 106 of the plurality of cell culture modules.

Referring back in the example embodiment of FIG. 1B, the means for capturing at least one image 226 may be configured to move back and forth in the first direction A according to the two-way arrow C between a first side 233 of the cell culture module 106 and a second side 234 of the cell culture module 106. The means for capturing at least one image 226 may be configured to move back and forth in the first direction A above and/or below the cell culture module 106. Each cell culture module 106 of the plurality of cell culture modules may have own imaging system comprising at least one means for capturing at least one image 226. According to an example embodiment, there is only one means for capturing at least one image 226 to capture at least one image from one culture module 106, which means for capturing at least one image 226 is moving between the first side 233 of the cell culture module 106 and the second side 234 of the cell culture module 106 above or below the cell culture modules. This means that the means for capturing images 226 may capture at least one image from the whole culture module 106 when it is moving from one side to another.

According to an example embodiment, the means for capturing at least one image 226 may be configured to move back and forth in the first direction A according to the two-way arrow C between the first side 229 of the cell culture plate 102 and the second side 230 of the cell culture plate 102, as can be seen in the example of FIG.1B. The cell culture plate 102 may comprise at least two cell culture modules 106 and may have own imaging system comprising at least one means for capturing at least one image 226. According to the example embodiment, there is one means for capturing at least one image 226 to capture at least one image from each of the at least two cell culture modules 106, which means for capturing at least one image 226 is moving between the first side 229 of the cell culture plate 102 and the second side 230 of the cell culture plate 102 above or below the at least two cell culture modules 106. This means that the means for capturing images 226 may capture at least one image from the whole culture plate 102 when it is moving from one side to another.

According to an example embodiment, each cell culture module 106 of the plurality of cell culture modules may comprise at least one means for capturing at least one image 226 from the membrane 214. The means for capturing images 226 may be arranged in the cavity 224.

According to the example embodiment, the plurality of cell culture modules 106 are configured to form at least one group 228, wherein the at least one group 228 comprise the at least one means for capturing at least one image 226. Each group 228 may comprise at least two cell culture modules 106 and the cell culture plate 102 may comprise one or more groups 228. The at least one means for capturing images 226 may capture the at least one image from at least part of the bottom of the at least one group 228 and/or from at least part of the top of the at least one group 228. Each group 228 may have own imaging system comprising at least one means for capturing at least one image 226. According to an example embodiment, there is one means for capturing at least one image 226 to capture at least one image from the group 228, which means for capturing at least one image 226 is moving between the first side 231 of the group 228 and the second side 232 of the group 288 above and/or below the group 228. This means that the means for capturing images 226 may capture at least one image from the whole group 228 when it is moving from one side to another. When the cell culture modules are divided in groups 228 less means for capturing images 226 may be needed.

According to an example embodiment, the at least one means for capturing at least one image 226 is compatible with at least one of the following: an upright and inverted microscope, a digital holography and confocal imaging system, and/or a multiwell plate reader.

According to an example embodiment, the means for capturing images 226 is configured to capture optical images. According to an example embodiment, the means for capturing images 226 comprises an optical fiber or an objective lens.

According to an example embodiment, the bottom part of the second chamber 208 and/or the at least two flow channels 210, 212 are made of transparent material.

According to an example embodiment, each of the at least two flow channels 210, 212 in at least one cell culture module 206 extends at a tilting angle α to the first direction A starting from the outlet 218, 222 of the corresponding culture medium reservoir 202, 204. The bottom part of the second chamber 208 and at least part of the each of the at least two flow channels 210, 212 may form the cavity 224.

In one embodiment, each of the second chamber 208, the first flow channel 210, and the second flow channel 212 may be coated, from inside, with a cell adhesion enhancing layer. The cell adhesion enhancing layer may be made of a hydrophilic material. Some non-restrictive examples of the hydrophilic material may include Matrigel, fibronectin, hyaluronic acid, different types of collagen (e.g., a type I collagen which is suitable for creating a blood-brain barrier cell culture model based on the apparatus 100), laminins, tenascin, elastane, nanocellulose with a host of different molecules. Such cell adhesion enhancing layers allow for less cells to be used in an experiment and more precise deposition of the cells on the basal surface of the membrane 214.

According to an example embodiment a layered structure represents one possible implementation example of the cell culture module 106. The layered structure may comprise a first layer and a second layer. The first layer may comprise the first and second culture medium reservoirs 202 and 204, as well as the first chamber 206. The second layer may comprise the second chamber 208 arranged exactly under the membrane 214, as well as the flow channels 210 and 212. The membrane 214 may be arranged between the first chamber 206 and the second chamber 208 to provide the fluid communication therebetween via the through pores. Moreover, the inlets of the flow channels 210 and 212 may be arranged exactly under the outlets 218 and 222 of the culture medium reservoirs 202 and 204, respectively, to provide the fluid communication between the reservoirs 202 and 204 and the flow channels 210 and 212, respectively. It should be noted that each of the first layer and the second layer may be made of room-temperature-vulcanizing (RTV) silicone (e.g., RTV615) or polydimethylsiloxane (PDMS). Moreover, the layers may be attached to each other by using adhesive or plasma bonding. The cell culture modules 106 thus configured may be attached to each other by the using the same adhesive or plasma bonding to implement the cell culture plate 102 of the apparatus 100.

According to a second embodiment, the cell culture module 106 may comprises a first culture medium reservoir 202, a second culture medium reservoir 204, a first (apical or top) chamber 206, a second (basal or bottom) chamber 208, a first flow channel 210, a second flow channel 212, a membrane 214, a cavity, and at least one means for capturing at least one image 226. The first culture medium reservoir 202 may have a top part with an inlet 216 for the culture medium and a bottom part with an outlet 218 for the culture medium. Similarly, the second culture medium reservoir 204 may have a top part with an inlet 220 for the culture medium and a bottom part with an outlet 222 for the culture medium. In general, the first culture medium reservoir 202, the second culture medium reservoir 204, the second chamber 208, the first flow channel 210, the second flow channel 12, the membrane 214, the cavity, and the means for capturing images 226 may be implemented in the same or similar manner as shown in FIGs 2A and 2B. However, the first chamber 206 may be implemented as a hollow tube having a curved or angled bottom. The curved or angled bottom of the first chamber 206 may comprise nine cavities each provided with one or more holes, so that the first chamber 206 and the second chamber 208 are in fluid communication via the through pores of the membrane 214. For example, there may be a single cavity with a single hole. In general, the configuration of the cavity or cavities depends on particular applications (e.g., a number of organoids and/spheroids to be formed on the apical surface of the membrane 214). According to an example embodiment, the curved or angled bottom of the first chamber 206 is coated, from outside, with a cell adhesion enhancing layer such that the holes of the cavities remain open. The cell adhesion enhancing layer may be the same as the one mentioned earlier in accordance with the first embodiment of the cell culture module 106.

Referring back to an example of FIG. 1A, the flow driving unit 104 is configured to cause the culture medium to flow, in each cell culture module 106 of the cell culture plate 102, between the first and second culture medium reservoirs 202 and 204. In one embodiment, the flow driving unit 104 may be a rocking platform that is configured to periodically rock the cell culture plate 102, thereby providing the culture medium flow. In another embodiment, the flow driving unit 104 may be a pneumatic pump that is configured to supply a compressed gas or a pressurized air to the inlet of each of the first and second culture medium reservoirs 202 and 204 in each cell culture module 106 of the cell culture plate 102, thereby causing the culture medium flow therein. In yet another embodiment, the flow driving unit 104 may be configured to cause the culture medium flow in each cell culture module 106 of the cell culture plate 102 by pipetting the culture medium from the first culture medium reservoir 202 to the second culture medium reservoir 204, or vice versa, at regular time intervals or based on a level of the culture medium in each of the reservoirs. Thus, unlike the current microfluidic cell culture devices, the apparatus 100 may be compatible with at least three different flow control methods which are indicated above.

In one embodiment, the apparatus 100 may further comprise a variety of electrodes. These electrodes may serve different purposes, and their arrangement inside the apparatus 100 depends on which purpose they are used for. In one embodiment, one or more cell culture modules of the cell culture plate 102 may comprise a first electrode arranged in the first chamber 206 and a second electrode arranged in the second chamber 208. In another embodiment, the first electrode may be arranged in the first culture medium reservoir 202 and/or the second culture medium reservoir 204 in one or more culture modules 106, while the second electrode may be arranged in the first chamber 206 of the cell culture module(s) 106. Both these embodiments with the first and second electrodes may be, for example, used to perform real-time Transepithelial/Transendothelial Electrical Resistance (TEER) measurements. In this case, the first and second electrodes may be connected to an external control unit.

Additionally or alternatively, the apparatus 100 may further comprise two or more third electrodes arranged in the first chamber 206 of one or more cell culture modules 106. These third electrodes may be arranged on the chamber walls for measuring a fluid level inside the first chamber 206. In this case, the third electrodes may either be in direct contact with the fluid/culture medium to determine the fluid level by performing conductivity measurements, or be embedded into the chamber walls (e.g., at a distance of 0.5 mm - 2 mm from each other) to determine the fluid level by measuring inductive or capacitive changes in the third electrodes. At the same time, the two third electrodes may be arranged opposite to each other such that one of the two third electrodes is used to issue an ultrasound wave and another of the two third electrodes is used to receive the ultrasound wave passed through the interior of the first chamber 206 (these ultrasound measurements may be performed just like radar measurements).

Additionally or alternatively, the apparatus 100 may further comprise two or more fourth electrodes embedded into the membrane 214 of one or more cell culture modules 106. These fourth electrodes may be used to provide different stimulus signals to cells deposited on the apical and/or basal surface of the membrane.

In one embodiment, the first chamber 206 or the second chamber 208 of one or more cell culture modules 106 of the cell culture plate 102 may further comprise an oxygen sensor, a pH sensor, a CO₂ sensor, or any combination thereof. These sensors may be used to enable real-time measurements of different parameters crucial for cell behavior.

FIG. 3 shows a block diagram of a cell culture incubator 600 in accordance with one exemplary embodiment. The cell culture incubator 600 may comprise the cell culture apparatus 100, and a pipetting station 602 coupled to the apparatus 100. More specifically, the pipetting station 602 may be configured to feed the culture medium to each of the first and second culture medium reservoirs 202 and 204 in each cell culture module 106 of the cell culture plate 102. The pipetting station 602 is well-known in this technical field, for which reason its description is omitted herein.

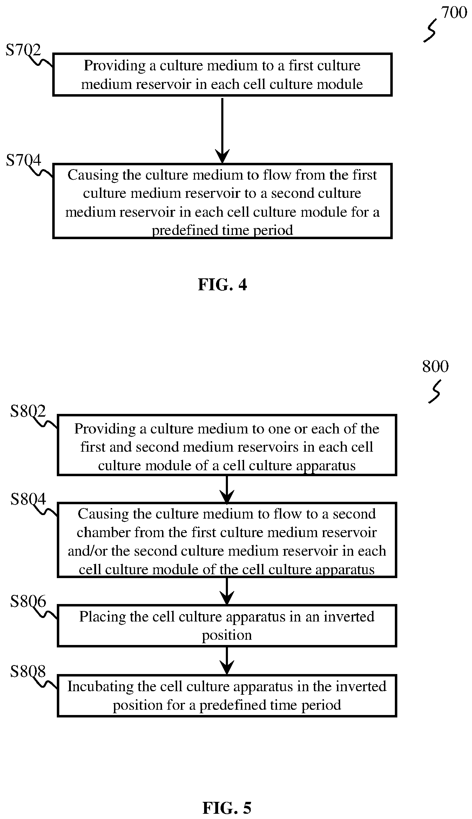

FIG. 4 shows a flowchart of a method 700 for cell cultivation by using the cell culture apparatus 100 in accordance with a first exemplary embodiment. The method 700 may start with an operation S702, in which a required culture medium is provided, e.g., by the pipetting station 602, to one of the first and second culture medium reservoirs 202 and 204 in each cell culture module 106 of the cell culture plate 102. Let us assume that the culture medium may be provided to the first culture medium reservoir 202. Then, the method 700 proceeds to an operation S704, in which the flow driving unit 104 may cause the culture medium to flow from the first culture medium reservoir 202 to the second culture medium reservoir 204 in each cell culture module 106 of the cell culture plate 102 for a predefined time period. The predefined time period may be selected based on the culture medium (i.e., a type of cells used therein). In particular, the cell deposition on the basal surface of the membrane 214 may be achieved if a flow rate is greater than gravity and a continuous flow lasts at least 30 min, preferably 60 min.

Types of liquid handling equipment may include pipets and micropipets, both digital and electronic, with fixed or disposable tips; microplate or microtiter plate dispensers, stackers, handlers, and washers; and a wide variety of automated robotic systems.

In one embodiment, the method 700 may comprise, during the operation S704, an additional operation, in which a different culture medium may be fed, e.g., by the pipetting station 602, via the first chamber 206 towards the membrane 214 in one or more cell culture modules 106 of the cell culture plate 102. If the first chamber 206 is used, a cell monolayer may be formed on the apical surface of the membrane 214, and this cell monolayer may interact with a cell layer forming on the basal surface of the membrane 214 via the through pores of the membrane 214. If the first chamber 206 is used, multiple microcavities may be bonded to the membrane 214, for which reason cells from the different culture medium may be trapped to form a single organoid in each cavity. In this case, the flow channels 210 and 212 may perfuse the culture medium through the membrane 214 to keep the organoids alive.

In one embodiment, the operation S704 of the method 700 may be performed by applying a positive pressure to the first and second culture medium reservoirs 210 and 212 in each cell culture module 106 of the cell culture plate 102 for the predefined time period (e.g., 60 min). In another embodiment, the operation S704 of the method 700 may be performed by applying a positive pressure to the first two culture medium reservoir 202 in each cell culture module 106 of the cell culture plate 102 for the predefined time period (e.g., 60 min), while capping the second culture medium reservoir 204, or vice versa. In yet another embodiment, the operation S704 of the method 700 may be performed by: (i) applying a first positive pressure to the first culture medium reservoir 202 in each cell culture module 106 of the cell culture plate 102 for the predefined time interval, while capping the second culture medium reservoir 204; and (ii) applying a second positive pressure to the second culture medium reservoir 204 in each cell culture module 106 of the cell culture plate 102 for the predefined time interval, while capping the first culture medium reservoir 202. The selection of each of the above-indicated embodiments of the operations S704 depends on particular applications.

According to an example embodiment, the method further comprises capturing at least one image from the membrane 214 by the at least one means for capturing at least one image 226 from at least part of a bottom of the at least one cell culture module and/or from at least part of a top of the at least one cell culture module. The images may be captured during any operation of method 700. The images may be taken when needed or on a scheduled basis as many as needed.

FIG. 5 shows a flowchart of a method 800 for cell cultivation by using the cell culture apparatus 100 in accordance with a second exemplary embodiment. The method 800 starts with an operation S802, in which a required culture medium may be provided to one or each of the first and second medium reservoirs 202 and 204 in each cell culture module 106 of the cell culture plate 102. Then, the method 800 proceeds to an operation S804, in which the flow driving unit 104 may cause the culture medium to flow to the second chamber 208 from the first medium culture reservoir 202 and/or the second medium reservoir 204 in each cell culture module 106 of the cell culture plate 102. After that, the method 800 goes on to an operation S806, in which the cell culture plate 102 may be placed in an inverted position. Next, the method 800 proceeds to an operation S808, in which the cell culture plate 102 may be incubated in the inverted position for a predefined time period. The predefined time period may be again selected based on the culture medium.

According to an example embodiment, similar to the method 700, the method 800 further comprises capturing at least one image from at least part of a bottom of the at least one cell culture module 106 and/or from at least part of a top of the at least one cell culture module 106 by the at least one means for capturing images 226. The images may be captured during any operation of method 800. The images may be taken when needed or on a scheduled basis as many as needed.

In one embodiment, similar to the method 700, the method 800 further comprises, during the operation S804, an additional operation, in which a different culture medium is fed, e.g., by the pipetting station 602, via the first chamber 206 towards the membrane 214 in one or more cell culture modules 106 of the cell culture plate 102. If the first chamber 206 is used, a cell monolayer may be formed on the apical surface of the membrane 214. If the first chamber 206 is used, a single organoid is formed in each cavity of the first chamber. The culture medium may be perfused through the flow channels 210 and 212 may comprise human brain vascular endothelial cells, while the different culture medium fed to the first chamber 206 may comprise human astrocytes. By using these types of cells, it is possible to mimic an artificial blood-brain barrier (BBB).

Further features of the method directly result for example from the functionalities and parameters of the cell culture apparatus 100 and the cell culture incubator 600 as described in the appended claims and throughout the specification and are therefore not repeated here. Different variations of the methods may be also applied, as described in connection with the various example embodiments.

A cell culture apparatus may be configured to perform or cause performance of any aspect of the methods described herein.

### Experimental results

The apparatus 100 comprising the cell culture modules like the one shown in FIGs. 2A and 2B was used in accordance with the method 800 to obtain BBB models based on mouse astrocytes and human brain endothelial cells. Each BBB model was obtained in two stages which are described below in more detail.

### I. Stage of preparing the apparatus 100:

### Materials:

| | |
|---|---|
| Extracellular Matrix (ECM) coating solution (used as a cell adhesion enhancing layer for the flow channels 210, 212, the second chamber 208, and the first chamber 206) | Collagen I (Col-I) 100 µg/mL, 0.1% acetic acid |
| Washing | 1xPBS (phosphate buffer saline), ethanol 96% |
| Plate 102 | 96-well format |

The stage of preparing was performed as follows:
1. The dry apparatus 100 was treated with plasma for 60 s.
2. The apparatus 100 was washed with 96% ethanol. 50 µL of 96 % ethanol was added to each open-top (first) chamber 206.
3. The ethanol inside the flow channels 210, 212 and the first chamber 206 was replaced/washed twice with 50 µL of PBS.
4. 50 µL of Col-I coating solution was injected into the flow channels 210, 212.
5. 50 µL of Col-I coating solution was added into each first chamber 206 of the apparatus 100.
6. Each culture medium reservoir was filled with 50 µL PBS, closed, and incubated at 37°C, 5 % CO₂, for 1 hour on the rocking platform (1 cycle per 15 min).
7. After incubation, the coating solution in the flow channels 210, 212 and each first chamber 206 of the apparatus 100 was replaced with PBS.

### II. Stage of obtaining the BBB model:

### Materials:

| | |
|---|---|
| Cells used for the BBB model (concentration, volume) | Mouse astrocytes (MA) (2 × 10⁶ cells/mL, min. 4.8 mL), mouse primary brain microvascular endothelial cells (MPBMEC) (2 × 10⁶ cells/mL, min. 4.8 mL). |
| | Human astrocytes (HA) (2 × 10⁶ cells/mL, min. 4.8mL), human primary brain microvascular endothelial cells (HPBMEC) (2 × 10⁶ cells/mL, min. 4.8mL). |

The BBB model based on the mouse cells (i.e., MA-cells and MPBMEC) was obtained as follows:
1. 4800 µL MA-cells at 2 × 10⁶ cells/mL were prepared according to the conventional cell culture protocols.
2. The MA-cells were placed on ice.
3. The MA-cell loading was performed by:
   a. removing all the PBS from the first chambers 206,
   b. replacing the PBS in the flow channels 210, 212 with µL 100 endothelial cell medium,
   c. pipetting 50 µL MA-cell suspension at 2 × 10⁶ cells/mL into the first chambers 206,
   d. incubating the apparatus 100 at 37°C, 5 % CO₂, for 1 hour (static, not on the rocking platform),
   e. checking every 10 min that the membrane 214 does not dry out (if required, a fresh culture medium may be added to the first chamber(s) 206 to keep the membrane 214 moist).
4. 4800 µL MPBMEC at 2 × 10⁶ cells/mL were prepared according to the conventional cell culture protocols.
5. The MPBMEC were again placed on ice.
6. The cell culture medium (added in the apparatus in step 3e) was replaced by injecting 50 µL MPBMEC suspension at 2 × 10⁶ cells/mL into the flow channels 210, 212 of the plate 102.
7. The plate 102 was manually rocked a few times to make sure the cell confluency is consistent.
8. The plate 102 was inverted and incubated at 37°C, 5 % CO₂, for 1 hour (static, not on the rocking platform).
9. 100 µL MPBMEC were added to the culture medium reservoir at one side of each flow channel in the plate 102.
10. 15 µL MPBMEC were added to the cell culture reservoir at one side of each flow channel in the plate 102.
11. The plate 102 was placed on the rocking platform at 37°C, 5 % CO₂.

The BBB model based on the human cells (i.e., HA-cells and HPBMEC) may be obtained in the same manner (one just needs to replace "MA" and "MPBMEC" with "HA" and "HPBMEC", respectively, in operations 1-11 above).

Thus, the MAs were introduced in the first chamber 206 and grown a monolayer on the apical surface of the membrane 214, while the MPBMEC were perfused through the second chamber 208 and deposited on the basal side of the membrane 214.

Subsequently, the cells were treated immunohistochemically with antibodies against CD31, glial fibrillary acidic protein (GFAP), Actin and DAPI (which is a small molecule that binds to DNA showing the cell nuclei).

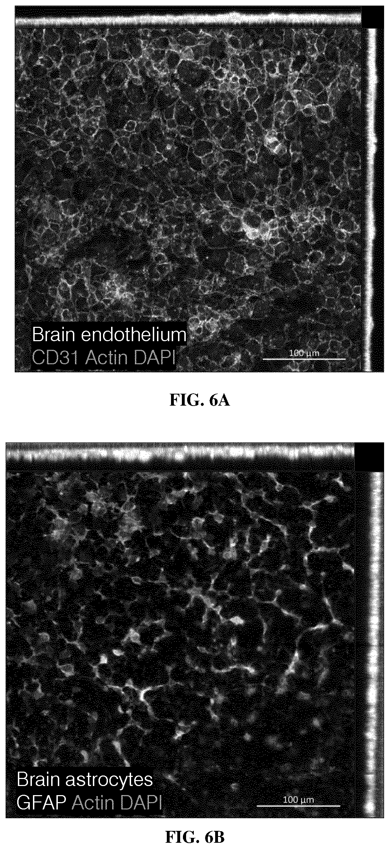

FIGs. 6A and 6B show confocal images of the BBB model by using the apparatus 100 in accordance with the method 800. More specifically, FIG. 6A shows the basal surface of the membrane 214, on which the MPBMEC were cultured. Adherensjunction marker CD31 (used as the cell type-specific marker of the MPBMEC) and actin expression have shown robust expression and indicated intact cell junctions. FIG. 6B shows the apical surface of the membrane (from the side of the first chamber 206), on which the MAs were cultured. FIG. 6B shows the GFAP (used as the cell type-specific marker of the mouse brain astrocytes) and actin expression.

Although the exemplary embodiments of the present disclosure are described herein, it should be noted that any various changes and modifications could be made in the embodiments of the present disclosure, without departing from the scope of legal protection which is defined by the appended claims. In the appended claims, the word "comprising" does not exclude other elements, steps or operations, and the indefinite article "a" or "an" does not exclude a plurality. The mere fact that certain measures are recited in mutually different dependent claims does not indicate that a combination of these measures cannot be used to advantage.

## Claims

1. A cell culture apparatus comprising:
a plurality of cell culture modules arranged adjacent to each other, each cell culture module of the plurality of cell culture modules comprising:
- at least two culture medium reservoirs each having a top part and a bottom part, the top part having an inlet for a culture medium and the bottom part having an outlet for the culture medium;
- a first chamber arranged between the at least two culture medium reservoirs such that the first chamber and the at least two culture medium reservoirs are aligned with each other in a first direction;
- a second chamber arranged under the first chamber and aligned with the first chamber in a second direction, the second direction being perpendicular to the first direction, the second chamber having a bottom part having at least two lateral holes, the bottom part of the second chamber being arranged higher than the bottom part of each of the at least two culture medium reservoirs;
- a membrane having through pores formed therein, the membrane being arranged between the first chamber and the second chamber such that the first chamber and the second chamber are in flow communication with each other via the through pores;
- at least two flow channels each connecting the outlet of the bottom part of one of the at least two culture medium reservoirs to one of the at least two lateral holes of the bottom part of the second chamber; and
- a cavity at least under the bottom part of the second chamber;
a flow driving unit configured to cause the culture medium to flow, in each cell culture module of the plurality of cell culture modules, between the at least two culture medium reservoirs via the at least two flow channels and the second chamber; and at least one means for capturing at least one image from at least part of a bottom of the at least one cell culture module and/or from at least part of a top of the at least one cell culture module.

2. The apparatus according to claim 1, wherein each of the at least two flow channels extends at least partly at a tilting angle to the first direction and wherein the bottom part of the second chamber and at least part of each of the at least two flow channels form a cavity.

3. The apparatus according to claim 1 or claim 2, wherein each of the at least two flow channels in at least one cell culture module comprising a first channel portion and a second channel portion, wherein
the first channel portion extending from the bottom part of one of the corresponding culture medium reservoirs and being parallel to the first direction; and
the second channel portion connecting the first channel portion to the one of the at least two lateral holes of the second chamber and extending at least partly at the tilting angle to the first direction; and
the bottom part of the second chamber and at least part of each second channel portions of the at least two flow channels form the cavity.

4. The apparatus according to claim 1 or claim 2, wherein each of the at least two flow channels in at least one cell culture module extends at the tilting angle to the first direction starting from the outlet of the corresponding culture medium reservoir and wherein the bottom part of the second chamber and each of the at least two flow channels form the cavity.

5. The apparatus according to any of any one of the preceding claims, wherein each of the at least two flow channels in at least one cell culture module of the plurality of cell culture modules have
a variable or constant channel height; and/or
a variable channel width which increases towards the second chamber.

6. The apparatus according to any of any one of the preceding claims, wherein the at least one means for capturing at least one image is configured to move back and forth in the first direction and capture the at least one image from at least part of the bottom of the at least one cell culture module and/orfrom at least part of the top of the at least one cell culture module.

7. The apparatus according to any of any one of the preceding claims, wherein at least two cell culture modules are configured to form at least one group, wherein the at least one means for capturing at least one image is configured to capture at least one image from at least part of the bottom of the at least two cell culture modules of the at least one group and/or from at least part of the top of the at least two cell culture modules of the at least one group.

8. The apparatus according to any one of claims 1 to 6, wherein the at least one means for capturing at least one image is configured to move back and forth in the first direction and capture the at least one image from at least part of the bottom of the cell culture module and/or from at least part of the top of the cell culture module.

9. The apparatus according to any of any one of the preceding claims, wherein the at least one means for capturing at least one image is arranged in the cavity

10. The apparatus according to any of any one of the preceding claims, wherein the at least one means for capturing at least one image is configured to capture at least one optical image.

11. The apparatus according to any of any one of the preceding claims, wherein the at least one means for capturing at least one image comprises an optical fiber or an objective lens.

12. The apparatus according to any of any one of the preceding claims, wherein the at least one means for capturing at least one image is compatible with at least one of the following: an upright and inverted microscope, a digital holography and confocal imaging system, and/or a multiwell plate reader.

13. A cell culture incubator comprising:
the cell culture apparatus according to any one of claims 1 to 12; and
a pipetting station configured to feed the culture medium to each of the at least two culture medium reservoirs in each cell culture module of the plurality of cell culture modules.

14. A method for cell cultivation by using the cell culture apparatus according to any one of claims 1 to 12, the method comprising:
(a) providing the culture medium to one of the at least two medium reservoirs in each cell culture module of the plurality of cell culture modules; and
(b) causing, by the flow driving unit, the culture medium to flow from said one of the at least two culture medium reservoirs to the rest of the at least two culture medium reservoirs in each cell culture module of the plurality of cell culture modules for a predefined time period, the predefined time period being selected based on the culture medium.

15. A method for cell cultivation by using the cell culture apparatus according to any one of claims 1 to 12, the method comprising:
(a) providing the culture medium to one or more of the at least two medium reservoirs in each cell culture module of the plurality of cell culture modules;
(b) causing, by the flow driving unit, the culture medium to flow to the second chamber from said one or more of the at least two medium reservoirs in each cell culture module of the plurality of cell culture modules;
(c) placing the cell culture apparatus in an inverted position; and
(d) incubating the cell culture apparatus in the inverted position for a predefined time period, the predefined time period being selected based on the culture medium.
